# EUROPEAN PATENT APPLICATION

(11) **EP 4 682 165 A1**
(43) Date of publication of application: **21.01.2026**
(21) Application number: 24771007.2
(22) Date of filing: 15.03.2024
(51) Int. Cl.: C07K 14/725, A61K 35/17, A61K 35/545, A61P 35/00, C12N 5/10, C12N 5/0783, C12N 15/63

(54) **T-CELL RECEPTOR FOR HPV-SPECIFIC CYTOTOXIC T-CELL, OR FUNCTIONAL FRAGMENT THEREOF**

(30) Priority: 16.03.2023 JP 2023042024
(71) Applicant: Juntendo Educational Foundation, Tokyo 113-8421 (JP)
(72) Inventor: ANDO, Miki, Tokyo 113-8421 (JP); ISHII, Midori, Tokyo 113-8421 (JP); ANDO, Jun, Tokyo 113-8421 (JP); NAKAUCHI, Hiromitsu, Tokyo 152-8550 (JP)
(74) Representative: Hoffmann Eitle
(86) International application number: PCT/JP2024/010248
(87) International publication number: WO 2024/190905

(57) **Abstract**

The invention provides a T-cell receptor or a functional fragment thereof having an excellent antitumor effect by cloning a T-cell receptor of an HPV 16E6-specific cytotoxic T cell.

A T-cell receptor or a functional fragment thereof of an HPV16E6-specific cytotoxic T cell, comprising an alpha chain CDR3 region consisting of the amino acid sequence set forth in SEQ ID NO: 3 or an amino acid sequence in which one to three amino acids are substituted, deleted or added to the amino acid sequence, and a beta chain CDR3 region consisting of the amino acid sequence set forth in SEQ ID NO: 8 or an amino acid sequence in which one to three amino acids are substituted, deleted or added to the amino acid sequence.

## Description

### TECHNICAL FIELD

The present invention relates to a T-cell receptor of an HPV-specific cytotoxic T cell, a functional fragment thereof, and use thereof.

### BACKGROUND ART

Cervical cancer contains high-risk human papillomavirus (HPV) in 99% of cases, with HPV16 accounting for 50-60% and HPV18 accounting for about 20%.

Prophylactic HPV vaccines can prevent initial infection by high-risk HPV types, but these vaccines are not effective against established cancer. In early stages, total hysterectomy is indicated, which results in loss of fertility even with ovarian preservation and increases the long-term risk of cardiovascular disease. Metastatic and recurrent cervical cancer is generally incurable. Furthermore, it is extremely resistant to chemotherapy, especially in young women (Non-Patent Literature 1).

As described above, most cervical cancers are intractable cancers caused by HPV infection, and there is no effective treatment method for advanced stages, so the development of new treatments is essential.

Since cervical cancer cells constitutively express E6 and E7 oncoproteins (factors contributing to tumorigenesis and cancer cell survival), E6 and E7 are attractive therapeutic targets.

### PRIOR ART DOCUMENTS

### NON-PATENT LITERATURE

Non-Patent Literature 1: Homepage of the Japan Society of Obstetrics and Gynecology, Public Interest Incorporated Association

### SUMMARY OF INVENTION

### PROBLEMS TO BE SOLVED BY THE INVENTION

An object of the present invention is to clone a T-cell receptor of an HPV-specific cytotoxic T cell and to provide a T-cell receptor or a functional fragment thereof having an excellent antitumor effect.

### SOLUTION TO THE PROBLEMS

Accordingly, the present inventors first induced HPV 16E6-CTLs targeting the HPV16 E6 antigen of cervical cancer from a healthy donor at a cell processing center, performed single-cell cloning, and then established T-iPS cells. After performing genome editing on HLA using CRISPR/Cas9 technology and carefully excluding off-target effects, a master cell bank was prepared and used as a raw material for clinical rejT cells. Subsequently, HPV16E6-rejT was differentiated and induced.

Furthermore, the present inventors succeeded in inducing HPV16E6-rejT containing a very large number of tissue-resident memory T cells, and proved that the binding of the surface antigen CD103 of the tissue-resident memory T cells to E-cadherin expressed on cervical cancer cells increases the production of cytotoxic proteins and increases antigen binding, thereby exhibiting a potent antitumor effect. By performing repertoire analysis on this HPV16E6-rejT rich in tissue-resident memory T cells, the full sequence of the T cell receptor (TCR) gene was confirmed, and the present invention was completed.

That is, the present invention provides the following inventions [1] to [26].
[1] A T-cell receptor or a functional fragment thereof of an HPV16E6-specific cytotoxic T cell, comprising an alpha chain CDR3 region consisting of the amino acid sequence set forth in SEQ ID NO: 3 or an amino acid sequence in which one to three amino acids are substituted, deleted or added to the amino acid sequence, and a beta chain CDR3 region consisting of the amino acid sequence set forth in SEQ ID NO: 8 or an amino acid sequence in which one to three amino acids are substituted, deleted or added to the amino acid sequence.
[2] The T-cell receptor or a functional fragment thereof according to [1], comprising an alpha chain CDR3 region consisting of the amino acid sequence set forth in SEQ ID NO: 3, and a beta chain CDR3 region consisting of the amino acid sequence set forth in SEQ ID NO: 8.
[3] The T-cell receptor or a functional fragment thereof according to [1] or [2], further comprising an alpha chain L region consisting of the amino acid sequence set forth in SEQ ID NO: 1 or an amino acid sequence in which one to three amino acids are substituted, deleted or added to the amino acid sequence, and a beta chain L region consisting of the amino acid sequence set forth in SEQ ID NO: 6 or an amino acid sequence in which one to three amino acids are substituted, deleted or added to the amino acid sequence.
[4] The T-cell receptor or a functional fragment thereof according to [1] or [2], further comprising an alpha chain L region consisting of the amino acid sequence set forth in SEQ ID NO: 1, and a beta chain L region consisting of the amino acid sequence set forth in SEQ ID NO: 6.
[5] The T-cell receptor or a functional fragment thereof according to any one of [1] to [4], further comprising an alpha chain V region consisting of the amino acid sequence set forth in SEQ ID NO: 2 or an amino acid sequence in which one to three amino acids are substituted, deleted or added to the amino acid sequence, and a beta chain V region consisting of the amino acid sequence set forth in SEQ ID NO: 7 or an amino acid sequence in which one to three amino acids are substituted, deleted or added to the amino acid sequence.
[6] The T-cell receptor or a functional fragment thereof according to any one of [1] to [4], further comprising an alpha chain V region consisting of the amino acid sequence set forth in SEQ ID NO: 2, and a beta chain V region consisting of the amino acid sequence set forth in SEQ ID NO: 7.
[7] The T-cell receptor or a functional fragment thereof according to any one of [1] to [6], further comprising an alpha chain J region consisting of the amino acid sequence set forth in SEQ ID NO: 4 or an amino acid sequence in which one to three amino acids are substituted, deleted or added to the amino acid sequence, and a beta chain J region consisting of the amino acid sequence set forth in SEQ ID NO: 9 or an amino acid sequence in which one to three amino acids are substituted, deleted or added to the amino acid sequence.
[8] The T-cell receptor or a functional fragment thereof according to any one of [1] to [6], further comprising an alpha chain V region consisting of the amino acid sequence set forth in SEQ ID NO: 4, and a beta chain J region consisting of the amino acid sequence set forth in SEQ ID NO: 9.
[9] A cell having the T-cell receptor or a functional fragment thereof according to any one of [1] to [8].
[10] The cell according to [9], which is an HPV16E6-specific cytotoxic T cell.
[11] An iPS cell having the T-cell receptor or a functional fragment thereof according to any one of [1] to [8].
[12] The iPS cell according to [11], which is an HPV16E6-specific cytotoxic T-iPS cell.
[13] A vector comprising DNA of the T-cell receptor or a functional fragment thereof according to any one of [1] to [8].
[14] A pharmaceutical composition comprising an HPV16E6-specific cytotoxic T cell or a T-iPS cell having the T-cell receptor or a functional fragment thereof according to any one of [1] to [8].
[15] The pharmaceutical composition according to [14], which is for use in treating cervical cancer.
[16] A cell-based drug comprising an HPV16E6-specific cytotoxic T cell having the T-cell receptor or a functional fragment thereof according to any one of [1] to [8].
[17] The cell-based drug according to [16], which is for use in treating cancer.
[18] Use of an HPV16E6-specific cytotoxic T cell or a T-iPS cell having the T-cell receptor or a functional fragment thereof according to any one of [1] to [8], for the manufacture of a drug for use in treating cervical cancer.
[19] An HPV16E6-specific cytotoxic T cell or a T-iPS cell having the T-cell receptor or a functional fragment thereof according to any one of [1] to [8], for the treatment of cervical cancer.
[20] A method for treating cervical cancer, comprising administering an HPV16E6-specific cytotoxic T cell or a T-iPS cell having the T-cell receptor or a functional fragment thereof according to any one of [1] to [8].
[21] An HPV16E6-specific cytotoxic T cell or a T-iPS cell, comprising a tissue-resident memory T cell.
[22] A pharmaceutical composition comprising an HPV16E6-specific cytotoxic T cell or a T-iPS cell, comprising a tissue-resident memory T cell.
[23] A cell-based drug comprising an HPV16E6-specific cytotoxic T cell or a T-iPS cell, comprising a tissue-resident memory T cell.
[24] An HPV16E6-specific cytotoxic T cell or a T-iPS cell, comprising a T cell whose surface antigens are CD3+, CD8+, CD62L-, CCR7-, CD103+ or CD69+.
[25] A pharmaceutical composition comprising an HPV16E6-specific cytotoxic T cell or a T-iPS cell, comprising a T cell whose surface antigens are CD3+, CD8+, CD62L-, CCR7-, CD103+ or CD69+.
[26] A cell-based drug comprising an HPV16E6-specific cytotoxic T cell or a T-iPS cell, comprising a T cell whose surface antigens are CD3+, CD8+, CD62L-, CCR7-, CD103+ or CD69+.

### EFFECTS OF THE INVENTION

The HPV-specific cytotoxic T cell or T-iPS cell having the T-cell receptor or a functional fragment thereof of the HPV16E6 antigen-specific cytotoxic T cell of the present invention is useful as a therapeutic agent for cervical cancer.

### BRIEF DESCRIPTION OF DRAWINGS

FIG. 1 shows the result of HPV16E6 tetramer staining after PBMC peptide stimulation.
FIG. 2 shows the result of HPV16E6 tetramer staining after single-cell cloning.
FIG. 3 shows the result of HPV16E6 tetramer staining of HPV16E6-rejT.
FIG. 4 shows the potent antigen recognition and cytotoxic effect of HPV16E6-rejT against cervical cancer cell lines (SiHa, SKGIIIa).
FIG. 5 shows that HPV16E6-rejT highly expresses Granzyme B and Perforin compared to an HPV16E6-CTL clone.
FIG. 6 shows that HPV16E6-rejT after HLA editing has an overwhelmingly higher proportion of tissue-resident memory T cells compared to an HPV16E6-CTL clone.
FIG. 7 shows that cervical cancer cells highly express E-cadherin, which is an epithelial marker.
FIG. 8 shows the amino acid sequences of the alpha and beta chains of the T-cell receptor of the HPV16E6-specific cytotoxic T cell.
FIG. 9 shows the base sequences encoding the amino acid sequences of the alpha and beta chains of the T-cell receptor of the HPV16E6-specific cytotoxic T cell.

### DESCRIPTION OF EMBODIMENTS

One aspect of the present invention is a T-cell receptor or a functional fragment thereof of an HPV16E6-specific cytotoxic T cell, characterized by having an alpha chain CDR3 region consisting of the amino acid sequence set forth in SEQ ID NO: 3 or an amino acid sequence in which one to three amino acids are substituted, deleted or added to the amino acid sequence, and a beta chain CDR3 region consisting of the amino acid sequence set forth in SEQ ID NO: 8 or an amino acid sequence in which one to three amino acids are substituted, deleted or added to the amino acid sequence.

Another aspect of the present invention is a T-cell receptor or a functional fragment thereof of an HPV 16E6-specific cytotoxic T cell, characterized by having an alpha chain CDR3 region consisting of the amino acid sequence set forth in SEQ ID NO: 3 or an amino acid sequence in which one to three amino acids are substituted, deleted or added to the amino acid sequence, and an alpha chain L region consisting of the amino acid sequence set forth in SEQ ID NO: 1 or an amino acid sequence in which one to three amino acids are substituted, deleted or added to the amino acid sequence; and a beta chain CDR3 region consisting of the amino acid sequence set forth in SEQ ID NO: 8 or an amino acid sequence in which one to three amino acids are substituted, deleted or added to the amino acid sequence, and a beta chain L region consisting of the amino acid sequence set forth in SEQ ID NO: 6 or an amino acid sequence in which one to three amino acids are substituted, deleted or added to the amino acid sequence.

Another aspect of the present invention is a T-cell receptor or a functional fragment thereof of an HPV 16E6-specific cytotoxic T cell, characterized by having an alpha chain CDR3 region consisting of the amino acid sequence set forth in SEQ ID NO: 3 or an amino acid sequence in which one to three amino acids are substituted, deleted or added to the amino acid sequence, an alpha chain L region consisting of the amino acid sequence set forth in SEQ ID NO: 1 or an amino acid sequence in which one to three amino acids are substituted, deleted or added to the amino acid sequence, and an alpha chain V region consisting of the amino acid sequence set forth in SEQ ID NO: 2 or an amino acid sequence in which one to three amino acids are substituted, deleted or added to the amino acid sequence; and a beta chain CDR3 region consisting of the amino acid sequence set forth in SEQ ID NO: 8 or an amino acid sequence in which one to three amino acids are substituted, deleted or added to the amino acid sequence, a beta chain L region consisting of the amino acid sequence set forth in SEQ ID NO: 6 or an amino acid sequence in which one to three amino acids are substituted, deleted or added to the amino acid sequence, and a beta chain V region consisting of the amino acid sequence set forth in SEQ ID NO: 7 or an amino acid sequence in which one to three amino acids are substituted, deleted or added to the amino acid sequence.

Another aspect of the present invention is a T-cell receptor or a functional fragment thereof of an HPV 16E6-specific cytotoxic T cell, characterized by having an alpha chain CDR3 region consisting of the amino acid sequence set forth in SEQ ID NO: 3 or an amino acid sequence in which one to three amino acids are substituted, deleted or added to the amino acid sequence, an alpha chain L region consisting of the amino acid sequence set forth in SEQ ID NO: 1 or an amino acid sequence in which one to three amino acids are substituted, deleted or added to the amino acid sequence, an alpha chain J region consisting of the amino acid sequence set forth in SEQ ID NO: 4 or an amino acid sequence in which one to three amino acids are substituted, deleted or added to the amino acid sequence, and an alpha chain V region consisting of the amino acid sequence set forth in SEQ ID NO: 2 or an amino acid sequence in which one to three amino acids are substituted, deleted or added to the amino acid sequence; and a beta chain CDR3 region consisting of the amino acid sequence set forth in SEQ ID NO: 8 or an amino acid sequence in which one to three amino acids are substituted, deleted or added to the amino acid sequence, a beta chain L region consisting of the amino acid sequence set forth in SEQ ID NO: 6 or an amino acid sequence in which one to three amino acids are substituted, deleted or added to the amino acid sequence, a beta chain V region consisting of the amino acid sequence set forth in SEQ ID NO: 7 or an amino acid sequence in which one to three amino acids are substituted, deleted or added to the amino acid sequence, and a beta chain J region consisting of the amino acid sequence set forth in SEQ ID NO: 9 or an amino acid sequence in which one to three amino acids are substituted, deleted or added to the amino acid sequence.

HPV is a type of virus belonging to the family Papillomaviridae and is deeply involved in the onset of cervical cancer. There are many types of HPV, but among the high-risk HPVs involved in the onset of cervical cancer, HPV16 is detected in 50-60% of cervical cancers. It is said that E6 and E7 of the HPV genes are deeply involved in the onset of cervical cancer.

The T-cell receptor (hereinafter referred to as TCR) is responsible for the antigen recognition function of T cells and is composed of proteins such as alpha chains, beta chains, gamma chains, and delta chains. Among these, a heterodimer of the TCR alpha chain protein (TCRα) and the TCR beta chain protein (TCRβ), or a heterodimer of gamma and delta chains, together with a CD3 complex (including gamma, delta, epsilon, and zeta) as an accessory molecule, CD4, or CD8, forms a TCR.

TCRα and TCRβ have a variable region (V region + J region) and a constant region (C region). Note that a complementarity-determining region (CDR) exists in the V region within the variable region. Therefore, a TCR can be characterized by the amino acid sequences of the V region (including the CDR region) or the V region and J region in TCRα and TCRβ.

As described above, the TCR of the present invention is characterized by having an alpha chain CDR3 region consisting of the amino acid sequence set forth in SEQ ID NO: 3 or an amino acid sequence in which one to three amino acids are substituted, deleted or added to the amino acid sequence, and a beta chain CDR3 region consisting of the amino acid sequence set forth in SEQ ID NO: 8 or an amino acid sequence in which one to three amino acids are substituted, deleted or added to the amino acid sequence.

Here, the amino acid sequence in which one to three amino acids are substituted, deleted or added to the amino acid sequence may be an amino acid sequence having an identity of 95% or more with the amino acid sequence, more preferably an amino acid sequence having an identity of 97% or more with the amino acid sequence, and even more preferably an amino acid sequence having an identity of 99% or more with the amino acid sequence.

The TCR of the present invention preferably has an alpha chain CDR3 region consisting of the amino acid sequence set forth in SEQ ID NO: 3, and a beta chain CDR3 region consisting of the amino acid sequence set forth in SEQ ID NO: 8.

In addition to the alpha chain CDR3 region and the beta chain CDR3 region, the TCR of the present invention preferably further has an alpha chain L region consisting of the amino acid sequence set forth in SEQ ID NO: 1 or an amino acid sequence in which one to three amino acids are substituted, deleted or added to the amino acid sequence, and a beta chain L region consisting of the amino acid sequence set forth in SEQ ID NO: 6 or an amino acid sequence in which one to three amino acids are substituted, deleted or added to the amino acid sequence.

Here, the amino acid sequence in which one to three amino acids are substituted, deleted or added to the amino acid sequence may be an amino acid sequence having an identity of 95% or more with the amino acid sequence, more preferably an amino acid sequence having an identity of 97% or more with the amino acid sequence, and even more preferably an amino acid sequence having an identity of 99% or more with the amino acid sequence.

The TCR of the present invention more preferably has an alpha chain CDR3 region consisting of the amino acid sequence set forth in SEQ ID NO: 3, an alpha chain L region consisting of the amino acid sequence set forth in SEQ ID NO: 1, a beta chain CDR3 region consisting of the amino acid sequence set forth in SEQ ID NO: 8, and a beta chain L region consisting of the amino acid sequence set forth in SEQ ID NO: 6.

In addition to the alpha chain CDR3 region, the alpha chain L region, the beta chain CDR3 region, and the beta chain L region, the TCR of the present invention more preferably has an alpha chain V region consisting of the amino acid sequence set forth in SEQ ID NO: 2 or an amino acid sequence in which one to three amino acids are substituted, deleted or added to the amino acid sequence, and a beta chain V region consisting of the amino acid sequence set forth in SEQ ID NO: 7 or an amino acid sequence in which one to three amino acids are substituted, deleted or added to the amino acid sequence.

Here, the amino acid sequence in which one to three amino acids are substituted, deleted or added to the amino acid sequence may be an amino acid sequence having an identity of 95% or more with the amino acid sequence, more preferably an amino acid sequence having an identity of 97% or more with the amino acid sequence, and even more preferably an amino acid sequence having an identity of 99% or more with the amino acid sequence.

The TCR of the present invention more preferably has an alpha chain CDR3 region consisting of the amino acid sequence set forth in SEQ ID NO: 3, an alpha chain L region consisting of the amino acid sequence set forth in SEQ ID NO: 1, and an alpha chain V region consisting of the amino acid sequence set forth in SEQ ID NO: 2; and a beta chain CDR3 region consisting of the amino acid sequence set forth in SEQ ID NO: 8, a beta chain L region consisting of the amino acid sequence set forth in SEQ ID NO: 6, and a beta chain V region consisting of the amino acid sequence set forth in SEQ ID NO: 7.

In addition to the alpha chain CDR3 region, the alpha chain L region, the beta chain CDR3 region, the alpha chain V region, the beta chain L region, and the beta chain V region, the TCR of the present invention more preferably has an alpha chain J region consisting of the amino acid sequence set forth in SEQ ID NO: 4 or an amino acid sequence in which one to three amino acids are substituted, deleted or added to the amino acid sequence, and a beta chain J region consisting of the amino acid sequence set forth in SEQ ID NO: 9 or an amino acid sequence in which one to three amino acids are substituted, deleted or added to the amino acid sequence.

Here, the amino acid sequence in which one to three amino acids are substituted, deleted or added to the amino acid sequence may be an amino acid sequence having an identity of 95% or more with the amino acid sequence, more preferably an amino acid sequence having an identity of 97% or more with the amino acid sequence, and even more preferably an amino acid sequence having an identity of 99% or more with the amino acid sequence.

The TCR of the present invention more preferably has an alpha chain CDR3 region consisting of the amino acid sequence set forth in SEQ ID NO: 3, an alpha chain L region consisting of the amino acid sequence set forth in SEQ ID NO: 1, an alpha chain V region consisting of the amino acid sequence set forth in SEQ ID NO: 2, an alpha chain J region consisting of the amino acid sequence set forth in SEQ ID NO: 4, a beta chain CDR3 region consisting of the amino acid sequence set forth in SEQ ID NO: 8, a beta chain L region consisting of the amino acid sequence set forth in SEQ ID NO: 6, a beta chain V region consisting of the amino acid sequence set forth in SEQ ID NO: 7, and a beta chain J region consisting of the amino acid sequence set forth in SEQ ID NO: 9.

Examples of the functional fragment of the TCR of the present invention include a polypeptide having the alpha chain CDR3 region and the beta chain CDR3 region, a polypeptide having the alpha chain CDR3 region, the alpha chain L region, the beta chain CDR3 region, and the beta chain L region, a polypeptide having the alpha chain CDR3 region, the alpha chain L region, and the alpha chain V region; and the beta chain CDR3 region, the beta chain L region, and the beta chain V region, a polypeptide having the alpha chain CDR3 region, the alpha chain L region, the alpha chain V region, and the alpha chain J region; and the beta chain CDR3 region, the beta chain L region, the beta chain V region, and the beta chain J region, and conjugates of these polypeptides.

The TCR of the present invention can be obtained, for example, by inducing HPV16E6-specific cytotoxic T cells from the peripheral blood of a patient or a healthy person, performing single-cell cloning, and establishing an HPV16E6-specific cytotoxic T cell clone.

To induce HPV16E6-specific cytotoxic T cells from peripheral blood, a healthy person or a cervical cancer patient may be used.

The T cell induced into an iPS cell in the present invention is preferably an HPV16E6-specific T cell. For example, it is a T cell that expresses CD3 and CD8, and specifically, a CTL that is a CD8-positive cell. Furthermore, for example, it is a T cell that expresses CD3 and CD4, and specifically, a T cell that is a CD4-positive cell. The antigen specificity in T cells is provided by a rearranged TCR gene that is antigen-specific. From the viewpoint of production efficiency, although not particularly limited, it is preferable to use an antigen-specific CD8-positive T cell as the human T cell to be induced into a T-iPS cell in order to obtain an antigen-specific CD8-positive cell. When performing immunotherapy, it is preferable that the human T cell differentiated from an iPS cell has the same or substantially the same antigen specificity as the human T cell induced into the iPS cell. In addition, T cells without antigen specificity are also included as T cells to induce T-iPS cells. Specifically, gene-modified T cells such as CAR T cells or TCR-T cells can be mentioned.

Such T cells can be isolated from human tissue by a known method. Examples of human tissue include tissues containing the T cells, such as peripheral blood, lymph nodes, bone marrow, thymus, spleen, umbilical cord blood, and lesional tissue. Among these, peripheral blood is preferable from the viewpoint of low invasiveness to the human body and easy preparation. When isolating tumor-infiltrating lymphocytes (TILs), they can be isolated from tumor tissue or peripheral blood. Known methods for isolating human T cells include, for example, magnetic selection using magnetic beads for cell separation, flow cytometry using an antibody against a cell surface marker such as CD4 or CD8 and a cell sorter, and an activated T cell induction method using an anti-CD3 antibody and an anti-CD28 antibody. In addition, desired T cells can be isolated using cytokine secretion, expression of functional molecules, or signal molecules such as PD-1 as indicators. Furthermore, cytotoxic T cells (CTLs) can be isolated using the secretion or production of granzyme or perforin as an indicator. In addition, when isolating from human tissue containing T cells with antigen specificity, T cells with desired antigen specificity can be purified from human tissue using a multimerized MHC (major histocompatibility complex) to which a desired antigen has been bound (for example, "MHC tetramer" or "Pro5^{®} MHC class I pentamer").

In the present invention, the genes introduced to make T cells into iPS cells are preferably a combination of at least four types of genes from among (a) an Oct3/4 gene, (b) a c-Myc gene, (c) a Sox2 gene, (d) a Klf4 gene, (e) a NANOG gene, and (f) a LIN28 gene.

In the present invention, the method for introducing the gene group into T cells is not particularly limited, and a known method can be appropriately selected and used. For example, in the case of introducing a nucleic acid encoding the gene group into T cells, a nucleic acid (for example, cDNA, RNA) encoding the gene group can be inserted into an appropriate expression vector containing a promoter that functions in T cells, and the expression vector can be introduced into cells by infection, lipofection method, liposome method, electroporation method, calcium phosphate co-precipitation method, DEAE-dextran method, microinjection method, or electroporation method.

Among such expression vectors, it is more preferable to use a stealth RNA expression vector containing the gene group, from the viewpoint of reducing the risk of oncogenesis and improving introduction efficiency. A stealth RNA expression vector is a vector designed to avoid entering chromosomes and to express genes persistently and stably in the cytoplasm rather than in the nucleus. It can introduce large genes of 13,000 base pairs or more, and can also introduce 10 genes simultaneously, does not damage cells, can be removed when the introduced genes are no longer needed, and has stealth properties that prevent the cells from recognizing the vector as a foreign object. Examples of such a stealth RNA expression vector include a complex composed of a minus single-stranded RNA (A) containing the following RNA sequences (1) to (8), a single-stranded RNA-binding protein (B), and an RNA-dependent RNA polymerase, wherein the complex does not activate the innate immune structure.
(1) An RNA sequence for the gene group,
(2) An RNA sequence derived from human mRNA that constitutes a non-coding region,
(3) A transcription start signal sequence recognized by the RNA-dependent RNA polymerase,
(4) A transcription termination signal sequence recognized by the RNA-dependent RNA polymerase,
(5) An RNA sequence containing a replication origin recognized by the RNA-dependent RNA polymerase,
(6) An RNA sequence encoding the RNA-dependent RNA polymerase,
(7) An RNA sequence encoding a protein that regulates the activity of the RNA-dependent RNA polymerase,
(8) An RNA sequence encoding the single-stranded RNA-binding protein.

In addition, when establishing T-iPS cells, it is preferable to activate the T cells by stimulating them with an anti-CD3 antibody and an anti-CD28 antibody in the presence of interleukin-2 (IL-2) or interleukin-7 (IL-7) and interleukin-15 (IL-15) before introducing the gene group. The T cells may also be stimulated and activated by at least one substance selected from the group consisting of phytohemagglutinin (PHA), interleukin-2 (IL-2), allogeneic antigen-expressing cells, an anti-CD3 antibody, an anti-CD28 antibody, and CD3 and CD28 agonists. Such stimulation can be performed, for example, by adding PHA, IL-2, an anti-CD3 antibody and/or an anti-CD28 antibody, etc. to the medium and culturing the T cells for a certain period of time. In addition, the anti-CD3 antibody and anti-CD28 antibody may be those to which magnetic beads or the like are bound, and furthermore, instead of adding these antibodies to the medium, stimulation can be given by culturing the T cells for a certain period of time on a culture dish with the anti-CD3 antibody and anti-CD28 antibody bound to the surface. Furthermore, stimulation may be given by adding an antigen peptide recognized by the T cells together with feeder cells to the medium.

The concentration of PHA to be added to the medium to give such stimulation to the T cells is not particularly limited, but is preferably 1 to 100 µg/mL. The concentration of IL-2 to be added to the medium is not particularly limited, but is preferably 1 to 200 ng/mL. Furthermore, the concentrations of the anti-CD3 antibody and the anti-CD28 antibody to be added to the medium are not particularly limited, but are preferably 1 to 10 times the culture volume of the T cells. The concentrations of the anti-CD3 antibody and the anti-CD28 antibody bound to the surface of the culture dish to give such stimulation to the T cells are not particularly limited, but the concentrations for coating are preferably 0.1 to 100 µg/mL for the anti-CD3 antibody, more preferably 1 to 100 µg/mL, and 0.1 to 10 µg/mL for the anti-CD28 antibody.

The culture period for performing such stimulation is not particularly limited as long as it is a period sufficient to give such stimulation to the T cells and to proliferate the T cells to the number of cells required for the introduction of the four genes, but it is usually 2 to 7 days, and is preferably 3 to 5 days from the viewpoint of gene transduction efficiency. From the viewpoint of infecting T cells by mixing them with the vector in a 15 mL tube or increasing gene transduction efficiency, it is preferable to culture them on a culture dish coated with Retronectin.

The medium for culturing the T cells and adding PHA, IL-2, an anti-CD3 antibody and/or an anti-CD28 antibody, etc. can be, for example, a known medium suitable for culturing the T cells, more specifically, Roswell Park Memorial Institute (RPMI) 1640 medium, AIM V^{™} medium, or NS-A2, which contains other cytokines and human serum. In addition to PHA, IL-2, an anti-CD3 antibody and/or an anti-CD28 antibody, the medium may contain amino acids (for example, L-glutamine) and antibiotics (for example, streptomycin, penicillin) necessary for culture. It is also preferable to add IL-7 and IL-15 to the medium instead of IL-2. The concentration of IL-7 and IL-15 to be added is not particularly limited, but is preferably 1 to 100 ng/mL for each.

In addition, the conditions for introducing the four genes into the T cells, or the subsequent conditions, are not particularly limited, but it is preferable to culture the T cells into which the four genes have been introduced under feeder-free conditions. Examples include wells coated with iMatrix-511 solution, which is a laminin 511 E8 fragment, or with vitronectin. The cells can be established by culture under feeder cell conditions, and examples of feeder cells include mouse embryonic fibroblasts (MEFs), STO cells, and SNL cells whose cell division has been stopped by radiation or antibiotic treatment.

Furthermore, in the process of inducing T-iPS cells from the T cells, it is preferable to add the iPS cell medium from the next day. After that, it is preferable to replace half of the medium every other day to gradually replace the T cell medium with the iPS cell medium.

It is also preferable to gradually replace the known medium suitable for culturing the T cells with a medium suitable for culturing iPS cells as the T cells transition to iPS cells, and to continue culturing. As a medium suitable for culturing iPS cells, a known medium can be appropriately selected and used, for example, StemFit AK03N when coated with iMatrix, Essential 8 Medium when coated with vitronectin, or Dulbecco's Modified Eagle Medium/F12 medium (human iPS cell medium) containing knockout serum replacement, L-glutamine, non-essential amino acids, 2-mercaptoethanol, and b-FGF on feeder cells such as MEF cells.

The selection of the T-iPS cells obtained in this way can be performed by appropriately selecting a known method. Examples of such a known method include a method of observing and selecting the morphology of ES cell/iPS cell-like colonies under a microscope. On the other hand, in the case of T-iPS cells established from a single-cell CTL clone, since the properties are often similar, there is also a method of not selecting each T-iPS cell colony and subculturing all of the established colonies as they are.

The confirmation that the selected cells are T-iPS cells can be performed, for example, by a method of detecting the expression of an undifferentiated cell-specific marker (ALP, SSEA-4, Tra-1-60, Tra-1-81, etc.) in the selected cells by immunostaining or RT-PCR, or by a method of transplanting the selected cells into a mouse and observing their teratoma formation. In addition, the confirmation that the selected cells are derived from the T cells can be performed by detecting the state of TCR gene rearrangement by genome PCR.

The timing of selecting and collecting these cells is preferably while observing the growth state of the colonies. Generally, it is 10 to 40 days, preferably 14 to 28 days, after introducing the gene group including the four genes into the T cells. Unless otherwise specified, the culture environment is preferably 5% CO₂, 35 to 38°C, and more preferably 37°C.

Next, HPV16E6-specific CTL cells are differentiated and induced from the established T-iPS cells.

The re-differentiation induction method is preferably a method of differentiating T-iPS cells into CD8+ single-positive T cells, and more preferably a method of differentiating T-iPS cells into CD4/CD8 double-negative T cells, and then differentiating the CD4/CD8 double-negative T cells into CD8+ single-positive T cells.

Furthermore, as described in Japanese Patent No. 6164746, it is preferable to obtain them by differentiating T-iPS cells into CD4/CD8 double-negative cells, stimulating the CD4/CD8 double-negative cells by adding a substance that stimulates the T-cell receptor, and then differentiating the CD4/CD8 double-negative cells stimulated with the T-cell receptor into CD8 single-positive T cells in the presence of the cytokines IL-7 and IL-15.

To differentiate T-iPS cells into CD4/CD8 double-negative cells, it is preferable to culture the T-iPS cells on feeder cells (preferably mouse stromal cells) in a medium containing cytokines, serum (for example, fetal bovine serum (FBS)), insulin, transferrin, sodium selenite, L-glutamine, α-monothioglycerol, ascorbic acid, etc..

The stromal cells to be used are preferably OP9 cells or 10T1/2 cells (C3H10T1/2 cells) that have been subjected to a treatment such as radiation. The cytokines added to the medium are preferably at least one cytokine selected from the group of VEGF, SCF, TPO, and FLT3L, and more preferably VEGF, SCF, and TPO, or VEGF, SCF, and FLT3L. Examples of the medium include X-VIVO medium, Iscove's Modified Dulbecco's Medium (IMDM medium), α-MEM, and DMEM, but IMDM medium is preferable from the viewpoint of making it easy to form T-iPS sacs (bag-like structures containing hematopoietic progenitor cells). The culture period for these T-iPS cells is preferably 8 to 14 days, and more preferably 10 to 14 days, after starting the culture of the T-iPS cells. The culture environment is not particularly limited, but is preferably 5% CO₂, 35 to 38°C, and more preferably 37°C. It is more preferable to culture for about one week under low oxygen concentration conditions (oxygen concentration: for example, 5 to 20%).

To differentiate T-iPS cells into CD4/CD8 double-negative cells, it is also preferable to culture the cells contained in the T-iPS sacs on feeder cells (preferably stromal cells, more preferably human stromal cells) in a medium containing cytokines, serum (for example, FBS), etc., but a cytokine-coated well is used under feeder-free conditions. The cells present inside the T-iPS sac can be separated, for example, by passing them through a sterilized sieve-like device (for example, a cell strainer, etc.). The stromal cells used for this culture are preferably OP9-DL1 cells, OP9-DL4 cells, 10T1/2/DL4 cells, or 10T1/2/DL1 cells that have been subjected to a treatment such as radiation, from the viewpoint of inducing differentiation into T cells via a notch signal. Examples of cytokines to be added to the medium include IL-7, FLT3L, VEGF, SCF, TPO, IL-2, and IL-15. Examples of the medium include α-MEM medium, DMEM medium, and IMDM medium, but α-MEM medium is preferable. In addition to IL-7 and FLT3L, the medium may contain amino acids (for example, L-glutamine) and antibiotics (for example, streptomycin, penicillin) necessary for culture.

The culture period for the cells contained in the T-iPS sacs is preferably a period until the T-cell receptor (TCR) is expressed on the cell surface of the CD4/CD8 double-negative cells obtained by differentiation in this way, and is preferably 14 to 28 days after starting the culture of the cells contained in the T-iPS sacs. The culture environment is not particularly limited, but is preferably 5% CO₂, 35 to 38°C, and more preferably 37°C.

Whether the T-cell receptor (TCR) is expressed on the cell surface of the CD4/CD8 double-negative cells can be evaluated by flow cytometry using an anti-TCRαβ antibody, an anti-CD3 antibody, an anti-CD4 antibody, and an anti-CD8 antibody.

In the method for manufacturing antigen-specific human CD8 single-positive cells having an antigen specificity, by stimulating the T-iPS cell-derived CD4/CD8 double-negative cells via the TCR expressed on the cell surface, further rearrangement of the TCRA gene can be suppressed, and consequently, the frequency of appearance of T cells having the same TCR gene rearrangement pattern as the original human T cells can be made extremely high in the CD8 single-positive cells obtained by re-differentiation.

The method for stimulating the T-cell receptor of the T-iPS cell-derived CD4/CD8 double-negative cells is preferably a method of bringing the T-iPS cell-derived CD4/CD8 double-negative cells into contact with at least one substance selected from the group consisting of an anti-CD3 antibody, an anti-CD28 antibody, an antigen peptide that specifically binds to the original human T cells of the T-iPS cells, a cell expressing a complex with HLA that shows constraint to the T-cell receptor, and an MHC multimer to which the antigen peptide is bound. From the viewpoint of giving physiological stimulation, a method of bringing them into contact with a specific peptide/HLA complex-expressing cell is more preferable. From the viewpoint of prioritizing the uniformity of stimulation, a method of bringing them into contact with an antibody or a reagent is more preferable.

The contact method can be performed, for example, by adding PHA, etc. to the medium and culturing the T cells for a certain period of time. In addition, the anti-CD3 antibody and anti-CD28 antibody may be those to which magnetic beads or the like are bound, and furthermore, instead of adding these antibodies to the medium, stimulation may be given by culturing the T cells for a certain period of time on a culture dish with the anti-CD3 antibody and anti-CD28 antibody bound to the surface. Furthermore, stimulation may be given by adding the antigen peptide together with feeder cells to the medium.

The concentration of PHA to be added to the medium to stimulate the TCR of the CD4/CD8 double-negative cells is preferably 1 to 100 µg/ml. The concentrations of the anti-CD3 antibody and the anti-CD28 antibody to be added to the medium are preferably 1 to 10 times the culture volume of the T cells. The concentrations of the anti-CD3 antibody and the anti-CD28 antibody bound to the surface of the culture dish to stimulate the TCR of the CD4/CD8 double-negative cells are preferably 0.1 to 100 µg/ml for the anti-CD3 antibody and 0.1 to 10 µg/ml for the anti-CD28 antibody for coating.

The culture period for the cells contained in the T-iPS sacs is preferably a period that includes the period necessary for the T-cell receptor (TCR) to be expressed on the cell surface of the CD4/CD8 double-negative cells obtained by differentiation in this way, and is preferably 7 to 29 days after starting the culture of the cells contained in the T-iPS sacs. The culture environment is preferably 5% CO₂, 35 to 38°C, and more preferably 37°C.

In the present invention, to differentiate the CD4/CD8 double-negative cells stimulated with the T-cell receptor into CD8 single-positive cells, it is preferable to culture the CD4/CD8 double-negative cells in a medium containing cytokines and serum (for example, human serum). The cytokines to be added to the medium may be any cytokines that can differentiate the CD4/CD8 double-negative cells into CD8 single-positive cells, and examples include IL-7 and IL-15. Among these, from the viewpoint of selecting the CD8 lineage and facilitating the generation of memory-type CD8+ T cells in the differentiation into CD8 single-positive cells, it is preferable to add a combination of IL-7 and IL-15. The concentrations of IL-7 and IL-15 to be added are preferably 1 to 20 ng/ml. Examples of the medium include RPMI-1640 medium, X-VIVO medium, DMEM medium, and α-MEM medium, but RPMI-1640 medium or X-VIVO medium is preferable. In addition to IL-7 and IL-15, the medium may also contain amino acids (for example, L-glutamine), antibiotics (for example, streptomycin, penicillin), and cytokines other than IL-7 and IL-15 necessary for culture.

In this culture, the CD4/CD8 double-negative cells may be co-cultured with feeder cells. The feeder cells are preferably peripheral blood mononuclear cells (PBMCs). The PBMCs are preferably in an allo (allogeneic) relationship with the CD4/CD8 double-negative cells. Furthermore, from the viewpoint of continuously stimulating the TCR and continuously suppressing further rearrangement of the TCR, it is more preferable to use peripheral blood mononuclear cells that present the antigen peptide that specifically binds to the original human T cells of the CD4/CD8 double-negative cells.

The culture period for differentiating the CD4/CD8 double-negative cells into CD8 single-positive cells is preferably 2 to 4 weeks. The culture environment is preferably 5% CO₂, 35 to 38°C, and more preferably 37°C.

The confirmation that the CD8 single-positive cells differentiated in this way are derived from T-iPS cells and also from the original T cells of the T-iPS cells can be performed, for example, by detecting the state of TCR gene rearrangement by genome PCR.

In addition, the CD8 single-positive cells obtained in this way can be isolated by appropriately selecting a known method. Examples of such a known method include flow cytometry using an antibody against the cell surface marker of CD8 and a cell sorter. For example, in the case of CD8 single-positive cells, a method of purification using an affinity column or the like on which an antigen recognized by the original T cells of the CD8 single-positive cells is immobilized, or a method of purification using an MHC multimer (for example, MHC tetramer) to which the antigen is bound can also be adopted.

Furthermore, the CD8 single-positive cells obtained according to the present invention do not express PD-1, but express CCR7 together with CD27 and CD28, which are representative of the central memory T cell phenotype, and the telomeres are longer compared to the original T cells, and they have high self-renewal ability. Accordingly, the present invention can produce CD8 single-positive T cells that have the same TCR gene rearrangement pattern as the original T cells, do not express PD-1, and express CD27, CD28, and CCR7. The T cells collected from a human differ from the obtained T cells in that they express PD-1 and the proportion of naive memory phenotype is small.

To maintain the CD8 single-positive cells obtained in this way, stimulation may be given to the cells every 1 to 2 weeks. Such stimulation includes contact with at least one substance selected from the group consisting of an anti-CD3 antibody, an anti-CD28 antibody, IL-2, IL-7, IL-15, an antigen recognized by the CD8 single-positive cells, an MHC multimer to which the antigen is bound, feeder cells in an allogeneic relationship with the CD8 single-positive cells, and feeder cells in an autologous relationship with the CD8 single-positive cells.

Whether the obtained iPS cell-derived T cells have HPV16E6-specific cytotoxic activity is confirmed by using an MHC pentamer, MHC tetramer, etc. to confirm that they retain the same antigen specificity as the original peripheral blood-derived CTLs. Furthermore, a TCR sequence analysis was performed to identify TCRαβ.

In addition, the HPV16E6-specific cytotoxic T cells can also be manufactured by genetic recombination using a gene encoding the TCR of the present invention. That is, for example, a gene encoding the TCR of the present invention may be introduced into a host T cell, and cells having HPV16E6-specific cytotoxic activity may be selected. To introduce the gene encoding the TCR into a host T cell, it is preferable to incorporate the gene into various viral vectors.

The selection of cells having HPV16E6-specific cytotoxic activity may be the same as the confirmation of HPV 16E6-specific cytotoxic activity described above, or it may be the detection of the ability to produce a cytokine such as IFN-γ.

The obtained HPV16E6-specific cytotoxic T cells or T-iPS cells are useful as a pharmaceutical composition or a cell-based drug.

Since the obtained HPV16E6-specific cytotoxic T cells or T-iPS cells have excellent HPV-specific cytotoxic activity, they are particularly useful as a therapeutic agent for cervical cancer.

Among the HPV16E6-specific cytotoxic T cells of the present invention, the HPV16E6-specific cytotoxic T cells rich in tissue-resident memory T cells have particularly excellent HPV-specific cytotoxic activity, and are therefore useful as a therapeutic agent for cervical cancer. It was confirmed that the HPV16E6-specific cytotoxic T cells rich in tissue-resident memory T cells exhibit a potent antitumor effect because the binding of the surface antigen CD103 of the tissue-resident memory T cells to E-cadherin expressed on cervical cancer cells increases the production of cytotoxic proteins and increases antigen binding.

Being rich in tissue-resident memory T cells means that the T cells, which are CD3+ and CD8+, are CD62L-, CCR7-, and CD103+ or CD69+. More preferably, it means that the T cells, which are CD3+ and CD8+, are CD62L-, CCR7-, CD103+, and CD69+. The proportion of tissue-resident memory T cells is preferably at least 30% or more, more preferably 50% or more, even more preferably 60% or more, and still more preferably 70% or more.

Another aspect of the present invention is a pharmaceutical composition for use in treating cervical cancer, comprising an HPV16E6-specific cytotoxic T cell or a T-iPS cell having the T-cell receptor or a functional fragment thereof.

Another aspect of the present invention is the use of an HPV16E6-specific cytotoxic T cell or a T-iPS cell having the T-cell receptor or a functional fragment thereof for the manufacture of a medicament for use in treating cervical cancer.

Another aspect of the present invention is an HPV16E6-specific cytotoxic T cell or a T-iPS cell having the T-cell receptor or a functional fragment thereof, for use in treating cervical cancer.

A further aspect of the present invention is a method for treating cervical cancer, characterized by administering an HPV16E6-specific cytotoxic T cell or a T-iPS cell having the T-cell receptor or a functional fragment thereof.

The pharmaceutical composition of the present invention may contain a pharmaceutically acceptable carrier in addition to the T cells of the present invention. Examples of such a carrier include physiological saline, Ringer's solution, etc. Furthermore, the pharmaceutical composition of the present invention may contain known pharmaceutically acceptable additives such as preservatives and coloring agents as necessary.

The form of the pharmaceutical composition of the present invention is preferably an injection, and an injection for use in T-cell infusion therapy is preferable. The pharmaceutical composition of the present invention is preferably administered to a cervical cancer patient by T-cell infusion at intervals of several weeks.

Furthermore, the pharmaceutical composition of the present invention is also useful for the treatment of HPV 16-infected cells and HPV 16-positive cells. It is particularly useful for the treatment of cancers caused by HPV16 infection, such as cancers of the anus, pharynx, vulva, and penis, in addition to cervical cancer.

### EXAMPLES

Next, the present invention will be described in more detail with reference to examples, but the present invention is not limited to these examples.

### Example 1

Establishment of T-iPS cells from an HPV16E6-specific CTL clone using a Sendai virus vector.
1) After isolating peripheral blood mononuclear cells from the peripheral blood of a healthy person, dendritic cells were induced for the purpose of antigen presentation. After 7 days, an antigen peptide (HPV16E6 49-57, A2402) was added to the induced dendritic cells and co-culture with peripheral blood mononuclear cells was started. After about 8 to 10 days, the CTLs were stained with an MHC tetramer to detect HPV 16E6-specific CTLs, and the tetramer positive rate was confirmed by flow cytometry. After confirming the HPV16E6-specific CTLs, single-cell sorting or tetramer/PE bead selection was performed, followed by a limiting dilution method.
2) After about 3 to 6 weeks, the established colonies were stained with a tetramer, and the establishment of the CTL clone was confirmed by flow cytometry. After confirming the establishment, the CTL clone was stimulated with CD3/28, and then genes were introduced using the following A) vector. The CTLs after gene introduction were transferred to a 6-well plate coated with iMatrix, and culture was started in a CO₂ incubator with CTL medium as the medium.
   A) SeV 6-factor vector ((a) Oct3/4 gene, (b) c-Myc gene, (c) Sox2 gene, (d) Klf4 gene, (e) NANOG gene, and (f) LIN28 gene).
3) The day after SeV gene introduction, an equal amount of iPS medium (StemFit AK03N) was added, and after that, half of the medium was replaced with StemFit AK03N every other day.
4) After 7 days, T-iPS cell colonies could be observed, and after that, colonies were picked up and expanded in culture.
5) The HLA of the established T-iPS cells was subjected to genome editing using CRISPR/Cas9 technology, and after carefully excluding off-target effects, a master cell bank was prepared.
6) After that, iPS cell-derived rejuvenated HPV16E6-CTL (HPV16E6-rejT) was differentiated and induced.
7) Cytotoxicity tests showed that the iPS cell-derived cells exhibited more potent antigen-specific cytotoxic activity against tumor cells than the original peripheral blood CTLs.

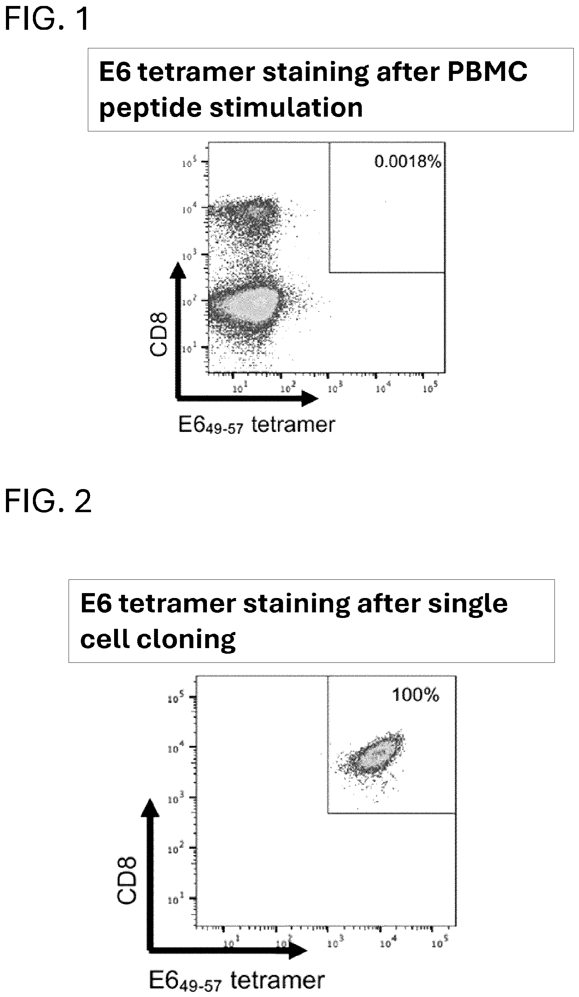

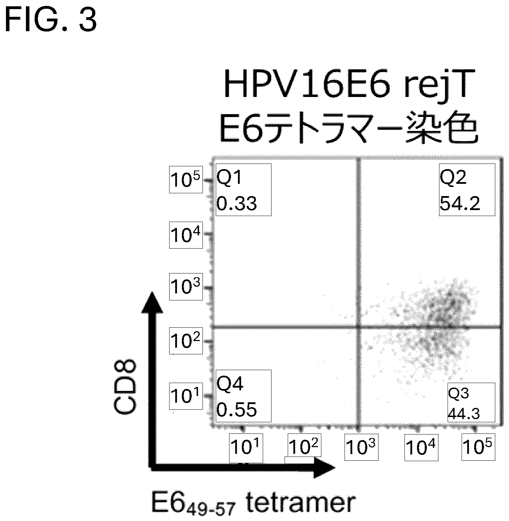

FIGS. 1 to 3 show the tetramer staining results of HPV 16E6-CTL induced from peripheral blood, the established HPV 16E6-specific CTL clone, and the iPS cell-derived HPV16E6-CTL (HPV16E6-rejT).

### Example 2

### 1) Cytotoxicity test

The cytotoxic activity of HPV16 E6-rejT after HLA editing and the HPV16E6-specific CTL clone against cervical cancer cell lines (SiHa, SKGIIIa) was compared by a chromium release assay.

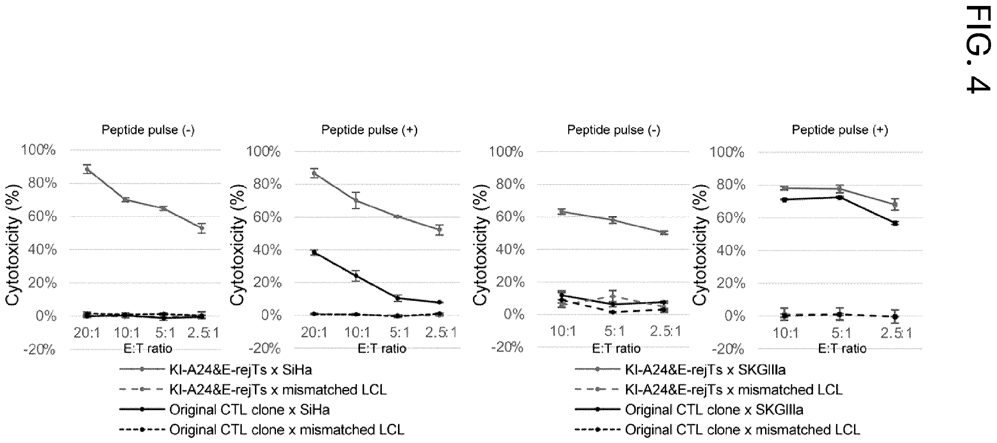

As shown in FIG. 4, when the HPV16 E6 antigen peptide was added to the target cervical cancer cell lines (SiHa, SKGIIIa), not only HPV16 E6-rejT after HLA editing but also the original peripheral blood-derived CTLs showed cytotoxic activity. However, when the HPV16 E6 antigen peptide was not added, only HPV16 E6-rejT after HLA editing showed cytotoxic activity. This confirmed the potent antigen recognition and cytotoxic effect of HPV16 E6-rejT after HLA editing against the cervical cancer cell lines (SiHa, SKGIIIa).

### 2) Characterization of HPV16E6-rejT after HLA editing

To confirm the expression of cytotoxic proteins of HPV16E6-rejT after HLA editing, intracellular staining for Granzyme B and Perforin was performed, and measured by flow cytometry.

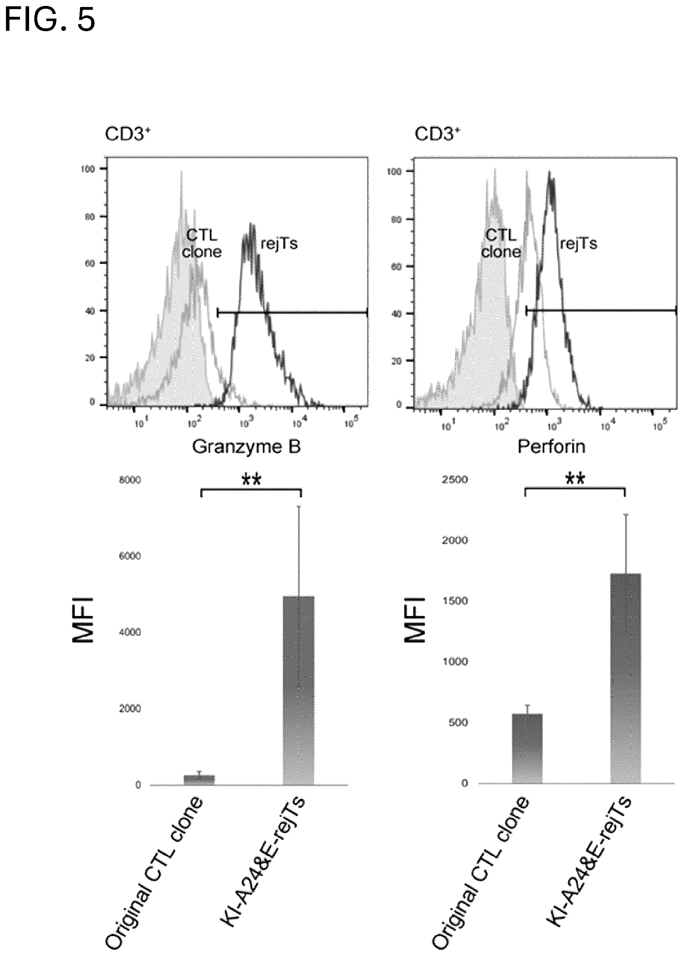

As shown in FIG. 5, it was revealed that HPV16E6-rejT after HLA editing highly expresses Granzyme B and Perforin compared to the HPV16E6-specific CTL clone.

### 3) Characteristics of HPV16E6-rejT after HLA editing

To confirm the proportion of tissue-resident memory T cells of HPV16E6-rejT after HLA editing, multiple staining was performed with CD3, CD8, CD62L, CCR7, CD27, CD103, and CD69, and measured by flow cytometry.

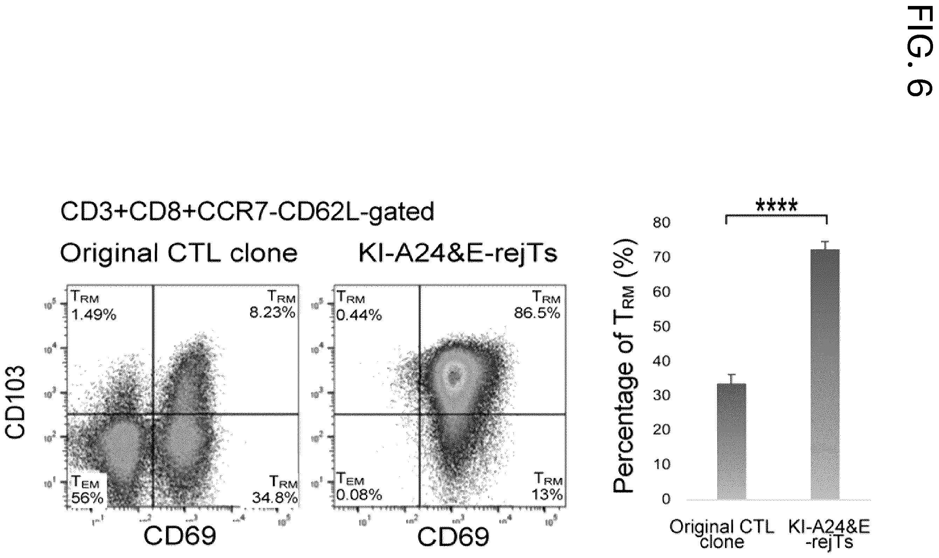

As shown in FIG. 6, it was revealed that HPV16E6-rejT after HLA editing has an overwhelmingly higher proportion of tissue-resident memory T cells compared to the HPV16E6-specific CTL clone.

### 4) Cervical cancer cells highly express E-cadherin, which is an epithelial marker.

To confirm that cervical cancer cells (SiHa, SKGIIIa, Caski) express E-cadherin, which is an epithelial marker, the surface antigens were stained with a dye-labeled antibody and measured by flow cytometry.

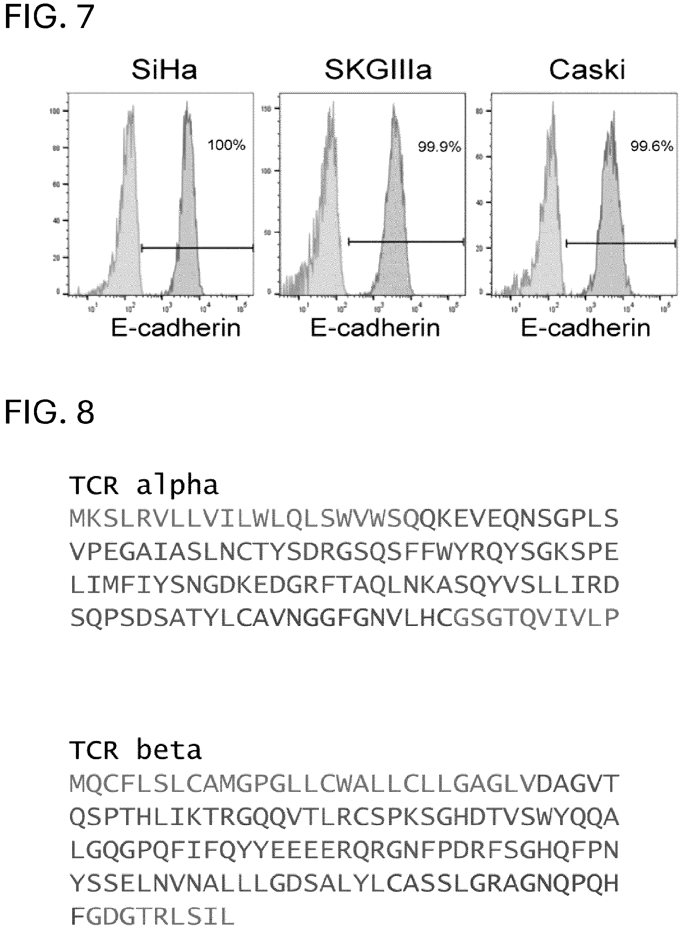

As shown in FIG. 7, it was found that cervical cancer cells (SiHa, SKGIIIa, Caski) highly express E-cadherin, which is an epithelial marker, and it was found that the HPV16E6-rejT rich in tissue-resident memory T cells of the present invention after HLA editing exhibits a potent antitumor effect and the binding of the surface antigen CD103 of the tissue-resident memory T cells to E-cadherin expressed on cervical cancer cells increases the production of cytotoxic proteins and increases antigen binding.

### Example 3

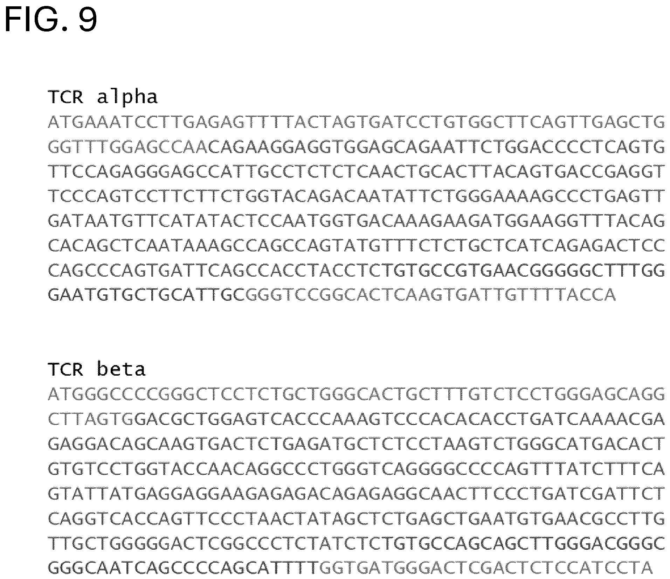

The results of the TCR sequence analysis are shown in FIG. 8 (amino acid sequence) and FIG. 9 (base sequence).

## Claims

1. A T-cell receptor or a functional fragment thereof of an HPV16E6-specific cytotoxic T cell, comprising an alpha chain CDR3 region consisting of the amino acid sequence set forth in SEQ ID NO: 3 or an amino acid sequence in which one to three amino acids are substituted, deleted or added to the amino acid sequence, and a beta chain CDR3 region consisting of the amino acid sequence set forth in SEQ ID NO: 8 or an amino acid sequence in which one to three amino acids are substituted, deleted or added to the amino acid sequence.

2. The T-cell receptor or a functional fragment thereof according to Claim 1, comprising an alpha chain CDR3 region consisting of the amino acid sequence set forth in SEQ ID NO: 3, and a beta chain CDR3 region consisting of the amino acid sequence set forth in SEQ ID NO: 8.

3. The T-cell receptor or a functional fragment thereof according to Claim 1, further comprising an alpha chain L region consisting of the amino acid sequence set forth in SEQ ID NO: 1 or an amino acid sequence in which one to three amino acids are substituted, deleted or added to the amino acid sequence, and a beta chain L region consisting of the amino acid sequence set forth in SEQ ID NO: 6 or an amino acid sequence in which one to three amino acids are substituted, deleted or added to the amino acid sequence.

4. The T-cell receptor or a functional fragment thereof according to Claim 1, further comprising an alpha chain L region consisting of the amino acid sequence set forth in SEQ ID NO: 1, and a beta chain L region consisting of the amino acid sequence set forth in SEQ ID NO: 6.

5. The T-cell receptor or a functional fragment thereof according to any one of Claims 1, further comprising an alpha chain V region consisting of the amino acid sequence set forth in SEQ ID NO: 2 or an amino acid sequence in which one to three amino acids are substituted, deleted or added to the amino acid sequence, and a beta chain V region consisting of the amino acid sequence set forth in SEQ ID NO: 7 or an amino acid sequence in which one to three amino acids are substituted, deleted or added to the amino acid sequence.

6. The T-cell receptor or a functional fragment thereof according to any one of Claims 1, further comprising an alpha chain V region consisting of the amino acid sequence set forth in SEQ ID NO: 2, and a beta chain V region consisting of the amino acid sequence set forth in SEQ ID NO: 7.

7. The T-cell receptor or a functional fragment thereof according to any one of Claims 1 to 6, further comprising an alpha chain J region consisting of the amino acid sequence set forth in SEQ ID NO: 4 or an amino acid sequence in which one to three amino acids are substituted, deleted or added to the amino acid sequence, and a beta chain J region consisting of the amino acid sequence set forth in SEQ ID NO: 9 or an amino acid sequence in which one to three amino acids are substituted, deleted or added to the amino acid sequence.

8. The T-cell receptor or a functional fragment thereof according to any one of Claims 1 to 6, further comprising an alpha chain V region consisting of the amino acid sequence set forth in SEQ ID NO: 4, and a beta chain J region consisting of the amino acid sequence set forth in SEQ ID NO: 9.

9. A cell having the T-cell receptor or a functional fragment thereof according to any one of Claims 1 to 8.

10. The cell according to Claim 9, which is an HPV16E6-specific cytotoxic T cell.

11. An iPS cell having the T-cell receptor or a functional fragment thereof according to any one of Claims 1 to 8.

12. The iPS cell according to Claim 11, which is an HPV16E6-specific cytotoxic T-iPS cell.

13. A vector comprising DNA of the T-cell receptor or a functional fragment thereof according to any one of Claims 1 to 8.

14. A pharmaceutical composition comprising an HPV 16E6-specific cytotoxic T cell or a T-iPS cell having the T-cell receptor or a functional fragment thereof according to any one of Claims 1 to 8.

15. The pharmaceutical composition according to Claim 14, which is for the treatment of cervical cancer.

16. A cell-based drug comprising an HPV16E6-specific cytotoxic T cell having the T-cell receptor or a functional fragment thereof according to any one of Claims 1 to 8.

17. The cell-based drug according to Claim 16, which is for use in treating cervical cancer.

18. Use of an HPV16E6-specific cytotoxic T cell or a T-iPS cell having the T-cell receptor or a functional fragment thereof according to any one of Claims 1 to 8, for the manufacture of a drug for use in treating cervical cancer.

19. An HPV16E6-specific cytotoxic T cell or a T-iPS cell having the T-cell receptor or a functional fragment thereof according to any one of Claims 1 to 8, for use in treating cervical cancer.

20. A method for treating cervical cancer, comprising administering an HPV16E6-specific cytotoxic T cell or a T-iPS cell having the T-cell receptor or a functional fragment thereof according to any one of Claims 1 to 8.

21. An HPV16E6-specific cytotoxic T cell or a T-iPS cell, comprising a tissue-resident memory T cell.

22. A pharmaceutical composition comprising an HPV 16E6-specific cytotoxic T cell or a T-iPS cell, comprising a tissue-resident memory T cell.

23. A cell-based drug comprising an HPV16E6-specific cytotoxic T cell or a T-iPS cell, comprising a tissue-resident memory T cell.

24. An HPV16E6-specific cytotoxic T cell or a T-iPS cell, comprising a T cell whose surface antigens are CD3+, CD8+, CD62L-, CCR7-, CD103+ or CD69+.

25. A pharmaceutical composition comprising an HPV 16E6-specific cytotoxic T cell or a T-iPS cell, comprising a T cell whose surface antigens are CD3+, CD8+, CD62L-, CCR7-, CD103+ or CD69+.

26. A cell-based drug comprising an HPV16E6-specific cytotoxic T cell or a T-iPS cell, comprising a T cell whose surface antigens are CD3+, CD8+, CD62L-, CCR7-, CD103+ or CD69+.
